# EUROPEAN PATENT APPLICATION

(11) **EP 4 603 890 A1**
(43) Date of publication of application: **20.08.2025**
(21) Application number: 23889043.8
(22) Date of filing: 02.11.2023
(51) Int. Cl.: G02B 27/01, G02B 27/00, G06V 40/18, G06F 3/01

(54) **HEAD-MOUNTED DISPLAY DEVICE FOR DISPLAYING TANGENT SCREEN AND OPERATION METHOD FOR HEAD-MOUNTED DISPLAY DEVICE**

(30) Priority: 08.11.2022 KR 20220148140
(71) Applicant: Samsung Electronics Co., Ltd., Suwon-si, Gyeonggi-do 16677 (KR)
(72) Inventor: CHO, Junghoon, Suwon-si, Gyeonggi-do 16677 (KR); KIM, Yongnam, Suwon-si, Gyeonggi-do 16677 (KR); PARK, Jisun, Suwon-si, Gyeonggi-do 16677 (KR); SOHN, Junil, Suwon-si, Gyeonggi-do 16677 (KR); YI, Sanghyun, Suwon-si, Gyeonggi-do 16677 (KR); KOO, Bonkon, Suwon-si, Gyeonggi-do 16677 (KR)
(74) Representative: Appleyard Lees IP LLP
(86) International application number: PCT/KR2023/017421
(87) International publication number: WO 2024/101781

(57) **Abstract**

The present disclosure includes a head-mounted display device and an operation method thereof, the head-mounted display device including a display configured to display a tangent screen for measuring a size and a shape of a scotoma, an eye-tracking sensor configured to obtain information about a gaze direction of a user, a memory storing at least one instruction, and at least one processor configured to execute the at least one instruction stored in the memory, wherein the at least one processor is further configured to identify a point of view where a gaze of the user intersects the display, based on the gaze direction of the user obtained via the eye-tracking sensor, determine a position on the display at which the tangent screen is to be displayed, such that a center of the tangent screen corresponds to the identified point of view, and display the tangent screen at the determined position.

## Description

### Technical Field

The present disclosure relates to a head-mounted display device and an operating method thereof.

### Background Art

Visual impairment may be generally classified into impairment associated with visual acuity, which refers to the clarity of vision a person possesses, and impairment associated with visual field, which refers to the extent of the external world visible to an eye gazing at one point straight ahead.

Macular degeneration, a type of visual field impairment, involves degeneration occurring in a macula, a part of the eye tissue, and when degeneration occurs in a macula, which is the central part of the retina, an intended focal point may appear dark or distorted, which may eventually lead to the formation of a scotoma, i.e., an area of lost vision within the central visual field, resulting in central vision loss.

When central vision impairment occurs due to macular degeneration, accurately identifying the size and shape of the scotoma is important for treatment, and in this case, an examination using a tangent screen may be performed to identify the size and shape of the scotoma.

Furthermore, in cases of central vision loss, objects may be perceived by using peripheral vision that has been trained through eccentric viewing training.

### Disclosure of Invention

### Solution to Problem

A head-mounted display device according to an embodiment of the present disclosure may include a display configured to display a tangent screen for measuring a size and a shape of a scotoma, an eye-tracking sensor configured to obtain information about a gaze direction of a user, a memory storing at least one instruction, and at least one processor configured to execute the at least one instruction stored in the memory. The at least one processor may identify a point of view where a gaze of the user intersects the display, based on the gaze direction of the user obtained via the eye-tracking sensor. The at least one processor may determine a position on the display at which the tangent screen is to be displayed, such that a center of the tangent screen corresponds to the identified point of view. The at least one processor may display the tangent screen at the determined position.

An embodiment of the present disclosure provides an operating method of a head-mounted display device including a display. In an embodiment of the present disclosure, the operating method of the head-mounted display device may include obtaining information about a gaze direction of a user viewing the display. In an embodiment of the present disclosure, the operating method of the head-mounted display device may include identifying, based on the obtained gaze direction of the user, a point of view where a gaze of the user intersects the display. In an embodiment of the present disclosure, the operating method of the head-mounted display device may include determining a position on the display at which a tangent screen is to be displayed, such that a center of the tangent screen for measuring a size and a shape of a scotoma of the user corresponds to the identified point of view. In an embodiment of the present disclosure, the operating method of the head-mounted display device may include displaying the tangent screen at the determined position.

An embodiment of the present disclosure may provide a computer-readable recording medium having recorded thereon a program for causing a computer to execute the operating method according to at least one of embodiments.

### Brief Description of Drawings

The present disclosure may be readily understood with a combination of the following detailed descriptions and the accompanying drawings, wherein reference numbers refer to structural elements.
FIG. 1 is a diagram for describing a head-mounted display device according to an embodiment of the present disclosure.
FIG. 2 is a block diagram illustrating a head-mounted display device and an operating method thereof, according to an embodiment of the present disclosure.
FIG. 3 is a diagram for describing an operation of measuring the size and shape of a scotoma of a user by using a tangent screen and at least one point, according to an embodiment of the present disclosure.
FIG. 4 is a diagram for describing a head-mounted display device and operations of the head-mounted display device, according to an embodiment of the present disclosure.
FIG. 5 is a flowchart for describing an operating method of a head-mounted display device according to an embodiment of the present disclosure.
FIG. 6 is a diagram for describing displaying a tangent screen on a display such that the center of the tangent screen corresponds to a point of view, according to an embodiment of the present disclosure.
FIG. 7 is a flowchart for describing a method of measuring the size and shape of a scotoma of a user by displaying a tangent screen and at least one point, according to an embodiment of the present disclosure.
FIG. 8A is a diagram for describing changing the position of at least one point according to a random pattern, according to an embodiment of the present disclosure.
FIG. 8B is a diagram for describing changing the position of at least one point according to a preset position pattern, according to an embodiment of the present disclosure.
FIG. 9 is a diagram for describing obtaining a notification signal for at least one point and measuring the size and shape of a scotoma of a user based on the obtained notification signal, according to an embodiment of the present disclosure.
FIG. 10 is a flowchart for describing a method of identifying, at preset tracking intervals, whether a gaze direction of a user has changed, and changing the position of a tangent screen, according to an embodiment of the present disclosure.
FIG. 11 is a diagram for describing changing, when a gaze direction of a user has changed, the position of a tangent screen such that the center of the tangent screen corresponds to a changed point of view, according to an embodiment of the present disclosure.
FIG. 12 is a flowchart for describing a method of, when a gaze direction of a user has changed, measuring the size and shape of a scotoma of the user while changing the position of a tangent screen, according to an embodiment of the present disclosure.
FIG. 13 is a diagram for describing displaying visual field training content on a display such that the center of the visual field training content corresponds to a point of view, according to an embodiment of the present disclosure.

### Mode for the Invention

Terms used herein will be briefly described, and then an embodiment of the present disclosure will be described in detail.

Although the terms used herein are selected from among common terms that are currently widely used in consideration of their functions in an embodiment of the present disclosure, the terms may be different according to an intention of one of ordinary skill in the art, a precedent, or the advent of new technology. Also, in particular cases, the terms are discretionally selected by the applicant of the present disclosure, in which case, the meaning of those terms will be described in detail in the corresponding description of an embodiment of the present disclosure. Therefore, the terms used herein are not merely designations of the terms, but the terms are defined based on the meaning of the terms and content throughout the present disclosure.

The singular expression may also include the plural meaning as long as it is not inconsistent with the context. All the terms used herein, including technical and scientific terms, may have the same meanings as those generally understood by those of skill in the art related to the present specification.

Throughout the present disclosure, when a part "includes" an element, it is to be understood that the part may additionally include other elements rather than excluding other elements as long as there is no particular opposing recitation. In addition, as used herein, the terms such as "...er (or)", "... unit", "... module", etc., denote a unit that performs at least one function or operation, which may be implemented as hardware or software or a combination thereof.

As used herein, the expression "configured to" may be interchangeably used with, for example, "suitable for", "having the capacity to", "designed to", "adapted to", "made to", or "capable of", according to a situation. The expression "configured to" may not imply only "specially designed to" in a hardware manner. Instead, in a certain circumstance, the expression "a system configured to" may indicate the system "capable of" together with another device or components. For example, "a processor configured (or set) to perform A, B, and C" may imply a dedicated processor (e.g., an embedded processor) for performing a corresponding operation or a generic-purpose processor (e.g., central processing unit (CPU) or an application processor) capable of performing corresponding operations by executing one or more software programs stored in a memory.

In addition, in the present disclosure, it should be understood that when components are "connected" or "coupled" to each other, the elements may be directly connected or coupled to each other, but may alternatively be connected or coupled to each other with an element therebetween, unless specified otherwise.

In the present disclosure, an 'electronic device' may be a head-mounted display (HMD) device. However, the present disclosure is not limited thereto, and the 'electronic device' may also be implemented as various types of electronic devices, such as a television (TV), a mobile device, a smart phone, a laptop computer, a desktop computer, a tablet PC, an electronic book terminal, a digital broadcasting terminal, personal digital assistant (PDA), a portable multimedia player (PMP), a wearable device, or the like.

In the present disclosure, a 'scotoma' may refer to an area in a central visual field that appears dark or distorted, or becomes invisible, resulting from degeneration occurring in the macula, which is a central part of the retina.

In the present disclosure, a 'tangent screen' is a planar perimeter that uses a plane to measure the visual field of a user gazing at the 'tangent screen', and is also referred to as a Bjerrum screen. The 'tangent screen' allows for the measurement of the size and shape of a scotoma in a user gazing at the center of the 'tangent screen'.

Hereinafter, embodiments of the present disclosure will be described in detail with reference to the accompanying drawings to allow those of skill in the art to easily carry out the embodiments. An embodiment of the present disclosure may, however, be embodied in many different forms and should not be construed as being limited to the embodiment of the present disclosure set forth herein. In addition, parts in the drawings unrelated to the detailed description are omitted to ensure clarity of an embodiment of the present disclosure, and like reference numerals in the drawings denote like elements.

Hereinafter, embodiments of the disclosure will be described in detail with reference to the drawings.

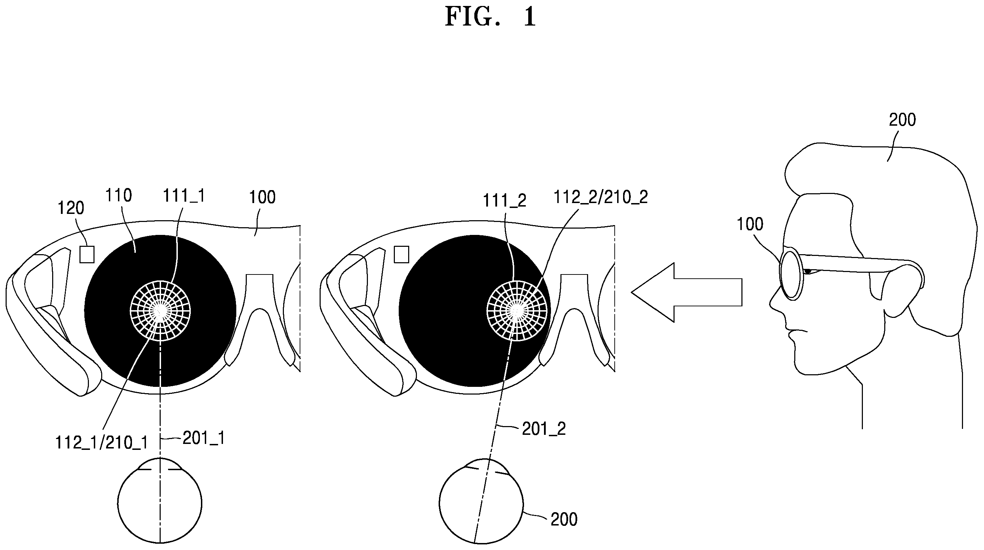

FIG. 1 is a diagram for describing an HMD device according to an embodiment of the present disclosure.

Referring to FIG. 1, an electronic device 100 according to an embodiment of the present disclosure may provide content to a user 200 wearing the electronic device 100. In an embodiment, the electronic device 100 is a device capable of providing content to the user 200, and may be an HMD device 100. In an embodiment, FIG. 1 illustrates that the electronic device 100 has a shape similar to eyeglasses, including a support portion that extends over a facial part of the user 200. However, the present disclosure is not limited thereto, and the electronic device 100 may also include a support portion that extends over a facial part and a head part of the user 200. In addition, in an embodiment of the present disclosure, the electronic device 100 is not limited to an HMD device and may also be implemented as various types of electronic devices, such as a TV, a mobile device, a smart phone, a laptop computer, a desktop computer, a tablet PC, an electronic book terminal, a digital broadcasting terminal, a PDA, a PMP, or a wearable device. Hereinafter, for convenience of description, the electronic device 100 will be referred to as the HMD device 100.

In an embodiment, the HMD device 100 may include a display 110 and an eye-tracking sensor 120 configured to obtain information about a gaze direction 201_1 or 201_2 of the user 200 viewing the display 110.

In an embodiment, the HMD device 100 may display, on the display 110, content obtained based on data received externally or data previously stored in the HMD device 100, so as to provide the content to the user 200. In an embodiment, the HMD device 100 may receive, from an external electronic device, data including information about content to be displayed on the display 110. However, the disclosure is not limited thereto. The HMD device 100 may be a video see-through electronic device capable of obtaining content by capturing, with a camera, a physical environmental space toward which the gaze direction of the user 200 is directed, and then providing the obtained content to the user US via the display 110.

Hereinafter, for convenience of description, the HMD device 100 will be described as a device capable of obtaining content based on data received externally or data previously stored in the HMD device 100, and displaying the obtained content on the display 110 to provide the content to the user 200.

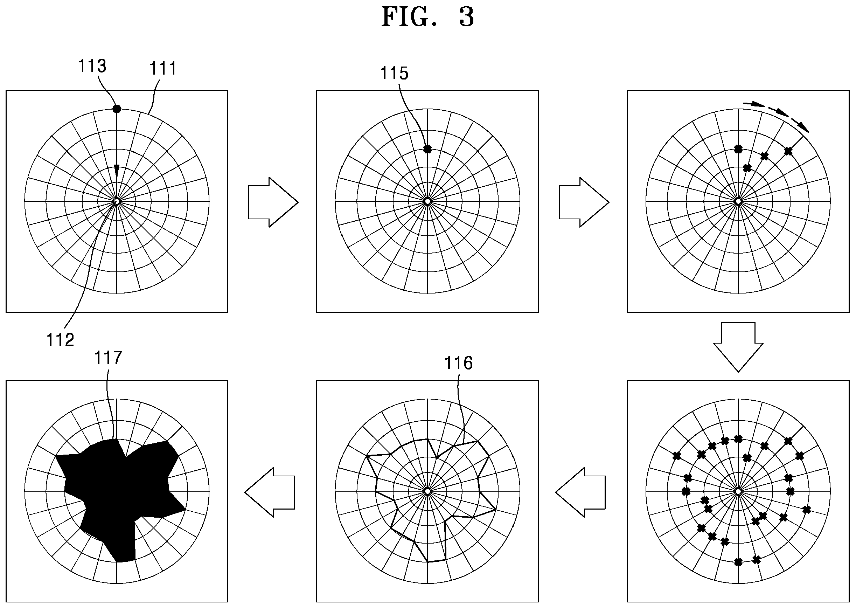

In an embodiment, the content displayed by the HMD device 100 on the display 110 may be a tangent screen 111_1 or 111_2 for measuring the size and shape of a scotoma 117 (see FIG. 3) of the user 200. In an embodiment, the content may also include at least one point 113 (see FIG. 3) displayed on the tangent screen 111_1 or 111_2. The tangent screens 111_1 and 111_2 and the at least one point 113 will be described below with reference to FIG. 3.

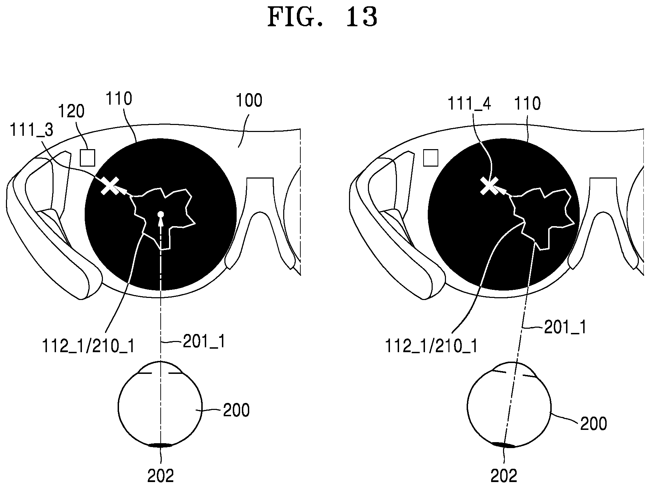

In an embodiment, the content displayed by the HMD device 100 on the display 110 may also include visual field training content 111_3 (see FIG. 13) for training the visual field of the user 200. The visual field training content 111_3 will be described below with reference to FIG. 13.

In an embodiment, the eye-tracking sensor 120 may track the gaze of an eye of the user 200 viewing the display 110, to obtain information about the gaze direction 201_1 or 201_2 of the user 200. In an embodiment, the eye-tracking sensor 120 may calculate the distance between an eye of the user 200 and the display 110. The HMD device 100 may identify, via the eye-tracking sensor 120, a point of view 210_1 or 210_2 where the gaze of the user 200 intersects the display 110. In an embodiment, when the gaze direction 201_1 or 201_2 of the user 200, obtained by the eye-tracking sensor 120, changes, the point of view 210_1 or 210_2, identified by the HMD device 100, where the gaze of the user 200 intersects the display 110, may also change.

In an embodiment, the HMD device 100 may display content on the display 110 such that a center 112_1 or 112_2 of the content corresponds to the point of view 210_1 or 210_2 where the obtained gaze of the user 200 intersects the display 110. In an embodiment, the HMD device 100 may display the tangent screens 111_1 and 111_2 on the display 110 such that the centers 112_1 and 112_2 of the tangent screens corresponds to the identified points of view 210_1 and 210_2, respectively. In an embodiment, the HMD device 100 may display the visual field training content 111_3 (see FIG. 13) on the display 110 such that a center 112_3 or 112_4 of the content corresponds to the identified point of view 210_1 or 210_2.

In an embodiment, the HMD device 100 may display the content on the display 110 such that the center 112_1 of the content corresponds to the identified point of view 210_1, and then determine whether the gaze direction 201_1 of the user 200 changes. When the gaze direction 201_1 of the user 200 changes, the HMD device 100 may identify, based on the changed gaze direction 201_2, the point of view 210_2 where the changed gaze of the user 200 intersects the display 110. The HMD device 100 may change the position where the content is displayed on the display 110, such that the center 112_2 of the content displayed on the display 110 corresponds to the point of view 210_2 identified based on the gaze of the user 200.

According to the HMD device 100 and an operating method of the HMD device 100 of the present disclosure, while measuring the size and shape of the scotoma 117 (see FIG. 3) of the user 200, the gaze direction 201_2 of the user 200 may change, such that the user 200 may no longer gaze at the center 112_1 of the content screen displayed on the display 110. In this case, the HMD device 100 may change the position of the content screen 111_2 displayed on the display 110, such that the size and shape of the scotoma 117 of the user is measured while maintaining the state in which the user gazes at the center 112_2 of the content screen. Thus, the accuracy and reliability of an operation of measuring the size and shape of the scotoma 117 of the user may be enhanced.

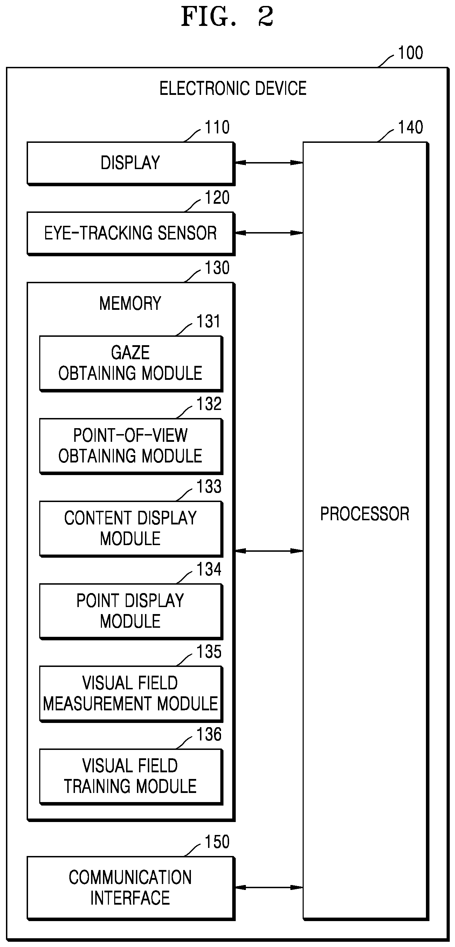

FIG. 2 is a block diagram illustrating an HMD device and an operating method thereof, according to an embodiment of the present disclosure.

Referring to FIG. 2, the HMD device 100 may include the display 110, the eye-tracking sensor 120, a memory 130, at least one processor 140, and a communication interface 150. However, not all components illustrated in FIG. 2 are essential components. The HMD device 100 may be implemented with more or fewer components than those illustrated in FIG. 2. In an embodiment, the HMD device 100 may further include a camera for photographing a real world space toward which the gaze direction of the user 200 is directed.

In an embodiment, the display 110, the eye-tracking sensor 120, the memory 130, the at least one processor 140, and the communication interface 150 may be electrically and/or physically connected to each other. Hereinafter, the same reference numerals are assigned to the same components as those described above with reference to FIG. 1B, and redundant descriptions thereof will be omitted.

In an embodiment, the display 110 may include any one of a liquid-crystal display, a plasma display, an organic light-emitting diode display, and an inorganic light-emitting diode display. However, the present disclosure is not limited thereto, and the display 110 may include other types of displays capable of providing content to the user 200.

Referring to FIGS. 1 and 2, in an embodiment, the eye-tracking sensor 120 may obtain information about the gaze direction 201_1 or 201_2 of the user 200. In an embodiment, the eye-tracking sensor 120 may detect the gaze direction 201_1 or 201_2 of the user 200 by detecting an image of an iris or pupil of the user 200, or by detecting the direction or amount of light, such as near-infrared light, reflected from a cornea. In an embodiment, the eye-tracking sensor 120 may include a left-eye eye-tracking sensor and a right-eye eye-tracking sensor. The eye-tracking sensor 120 may detect a gaze direction of the left eye of the user 200 and a gaze direction of the right eye of the user 200. Detecting the gaze direction 201_1 or 201_2 of the user 200 may include obtaining information about the gaze direction 201_1 or 201_2 of the user 200.

In an embodiment, the eye-tracking sensor 120 may also calculate the distance between the display 110 and an iris or pupil of the user 200 based on a preset positional relationship between the display 110 and the eye-tracking sensor 120, an image of the iris or pupil of the user 200, or the like. The eye-tracking sensor 120 may identify the point of view 210_1 or 210_2 where the gaze of the user 200 intersects the display 110, based on the gaze direction 201_1 or 201_2 of the user 200 and the distance between the display 110 and the iris of the user 200.

In an embodiment, the eye-tracking sensor 120 may include a light emission unit and a light detection unit. In an embodiment, the light emission unit may include an infrared (IR) light-emitting diode (LED) configured to emit infrared light toward an eye of the user 200, and the light detection unit may include an IR camera configured to detect light reflected from the eye. However, the present disclosure is not limited thereto. The light emission unit may include an IR scanner (e.g., a microelectromechanical systems (MEMS) scanner) configured to emit line-shaped IR light toward an eye of the user 200, and the light detection unit may include an IR detector (e.g., a photodiode) configured to detect light reflected from the eye of the user 200. In an embodiment, the left-eye eye-tracking sensor and the right-eye eye-tracking sensor may each include a light emission unit and a light detection unit.

In an embodiment of the present disclosure, the memory 130 may include at least one of flash memory-type memory, hard disk-type memory, multimedia card micro-type memory, card-type memory (e.g., SD or XD memory), random-access memory (RAM), static RAM (SRAM), read-only memory (ROM), electrically erasable programmable ROM (EEPROM), programmable ROM (PROM), mask ROM, flash ROM, a hard disk drive (HDD), or a solid-state drive (SSD). At least one instruction or program code for performing functions or operations of the HMD device 100 may be stored in the memory 130. At least one instruction, algorithm, data structure, program code, and application program stored in the memory 130 may be implemented in a programming or scripting language such as C, C++, Java, or an assembler.

In an embodiment, a gaze obtaining module 131, a point-of-view obtaining module 132, a content display module 133, a point display module 134, a visual field measurement module 135, and a visual field training module 136 may be stored in the memory 130. However, not all modules illustrated in FIG. 2 are essential modules. More or fewer modules than those illustrated in FIG. 2 may be stored in the memory 130.

A 'module' included in the memory 130 may refer to a unit that processes a function or operation performed by the at least one processor 140. A 'module' included in the memory 130 may be implemented as software, such as at least one instruction, algorithm, data structure, or program code.

Referring to FIGS. 1 and 2, in an embodiment, the gaze obtaining module 131 may include instructions or program code related to an operation or function for the HMD device 100 to obtain information about the gaze direction 201_1 or 201_2 of the user 200. In an embodiment, by executing the instructions or program code of the gaze obtaining module 131, the at least one processor 140 may obtain information about the gaze direction 201_1 or 201_2 of the user 200, via the eye-tracking sensor 120. However, the present disclosure is not limited thereto, and by executing instructions or program code of the gaze obtaining module 131, the at least one processor 140 may also obtain information about the gaze direction 201_1 or 201_2 of the user 200, from a server or a peripheral electronic device.

In an embodiment, the point-of-view obtaining module 132 may include instructions or program code related to an operation or function for identifying the point of view 210_1 or 210_2 where the gaze of the user 200 intersects the display 110, based on the obtained gaze direction 201_1 or 201_2 of the user 200. In an embodiment, by executing the instructions or program code of the point-of-view obtaining module 132, the at least one processor 140 may identify the point of view 210_1 or 210_2 where the gaze of the user 200 intersects the display 110, via the eye-tracking sensor 120.

In an embodiment, the content display module 133 may include instructions or program code related to an operation or function for determining a position on the display 110 where content is to be displayed, such that the center 112_1, 112_2, 112_3, or 112_4 (see FIG. 13) of the content corresponds to the identified point of view 210_1 or 210_2, and for displaying the content at the determined position. In an embodiment, by executing the instructions or program code of the content display module 133, the at least one processor 140 may determine a position on the display 110 where the content is to be displayed, such that the center 112_1, 112_2, 112_3, or 112_4 (see FIG. 13) of the content corresponds to the identified point of view 210_1 or 210_2. By executing the instructions or program code of the content display module 133, the at least one processor 140 may display the content at the determined position.

In an embodiment, the content that the at least one processor 140 displays on the display 110 by executing the instructions or program code of the content display module 133 may include the tangent screen 111_1 or 111_2 for measuring the size and shape of a scotoma of the user 200, and the visual field training content 111_3 for training the visual field of the user 200.

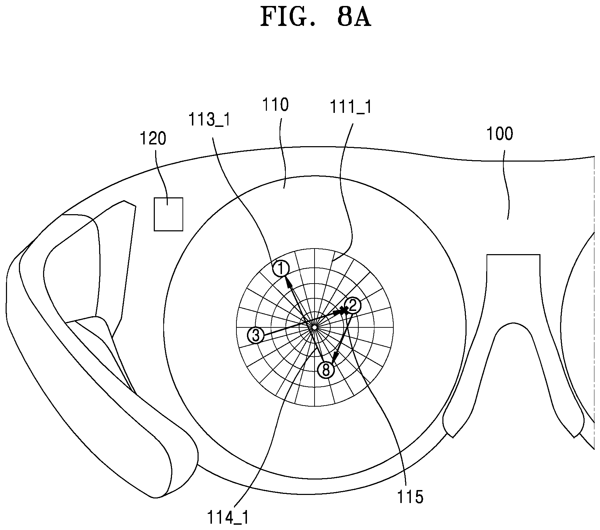

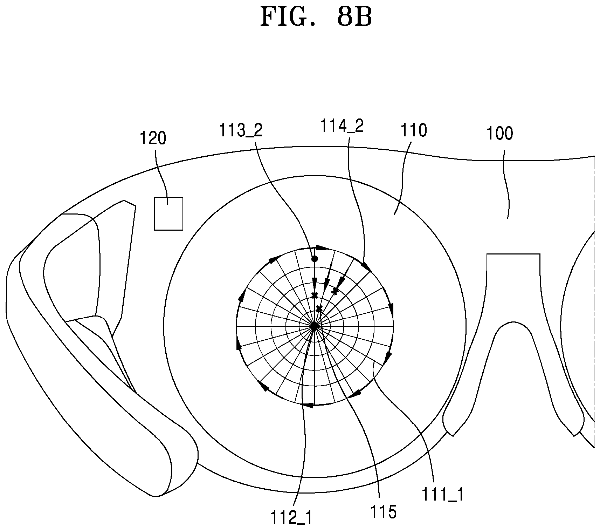

In an embodiment, the point display module 134 may include instructions or program code related to an operation or function for displaying at least one point 113_1 or 113_2 (see FIGS. 8A and 8B) on the tangent screen 111_1 or 111_2 when the tangent screen 111_1 or 111_2 is displayed on the display 110. In an embodiment, the point display module 134 may include instructions or program code related to an operation or function for changing the at least one point 113_1 or 113_2 according to a random pattern 114_1 (see FIG. 8A) or a preset position pattern 114_2 (see FIG. 8B), and displaying the changed at least one point 113_1 or 113_2 on the tangent screen 111_1 or 111_2. In addition, the point display module 134 may include instructions or program code related to an operation or function for changing the shape of the at least one point 113_1 or 113_2 to correspond to any one of a plurality of preset shapes and displaying the changed at least one point 113_1 or 113_2 on the tangent screen 111_1 or 111_2.

In an embodiment, by executing the instructions or program code of the point display module 134, the at least one processor 140 may display the at least one point 113_1 or 113_2 on the tangent screen 111_1 or 111_2. The at least one point 113_1 and 113_2 displayed on the tangent screens 111_1 and 111_2 of the present disclosure will be described below with reference to FIGS. 3, 8A, 8B, and 9.

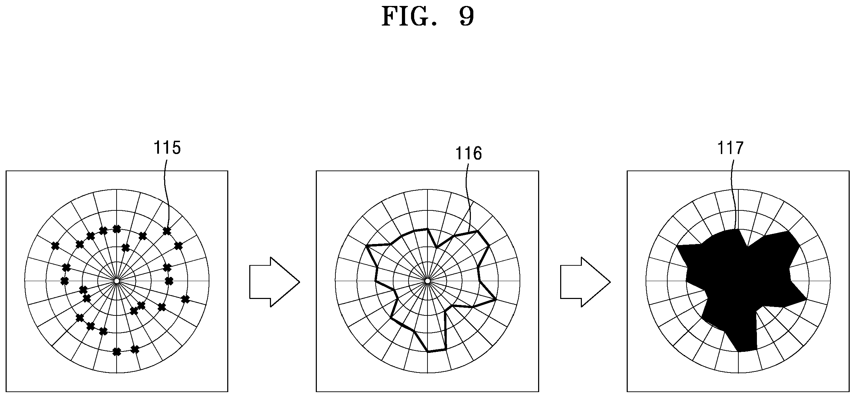

In an embodiment, the visual field measurement module 135 may include instructions or program code related to an operation or function for measuring the visual field of the user 200 via the tangent screen 111_1 or 111_2 and the at least one point 113_1 or 113_2 that are displayed on the display 110. In an embodiment, the visual field measurement module 135 may include instructions or program code related to an operation or function for measuring the size and shape of the scotoma 117 (see FIG. 9) of the user 200 via the tangent screen 111_1 or 111_2 and the at least one point 113_1 or 113_2 that are displayed on the display 110, and thereby measuring the visual field of the user 200.

In an embodiment, the visual field measurement module 135 may include instructions or program code related to an operation or function for receiving, from the user 200, a user input including information indicating that the at least one point 113_1 or 113_2 displayed on the tangent screen 111_1 or 111_2 is perceived abnormally, and for measuring the size and shape of the scotoma 117 of the user 200 based on the received user input.

In an embodiment, by executing the instructions or program code of the visual field measurement module 135, the at least one processor 140 may measure the size and shape of the scotoma 117 of the user 200 and measure the visual field of the user 200. Measuring the size and shape of the scotoma 117 of the user 200 according to the present disclosure will be described below with reference to FIGS. 3, 8A, 8B, and 9.

In an embodiment, the visual field training module 136 may include instructions or program code related to an operation or function for displaying, on the display 110, the visual field training content 111_3 for training the visual field of the user 200 measured via the visual field measurement module 135. In an embodiment, the visual field training module 136 may include instructions or program code related to an operation or function for displaying, on the display, the visual field training content 111_3 for performing, based on the measured size and shape of the scotoma 117 of the user 200, eccentric viewing training such that the user 200 may perceive objects through peripheral vision.

In an embodiment, the visual field training module 136 may include instructions or program code related to an operation or function for determining a position on the display 110 where the visual field training content 111_3 is displayed, such that the center 112_3 or 112_4 of the visual field training content 111_3 corresponds to the identified point of view 210_1 or 210_2, and for displaying the visual field training content 111_3 at the determined position.

In an embodiment, by executing the instructions or program code of the visual field training module 136, the at least one processor 140 may determine a position on the display 110 where the visual field training content 111_3 is to be displayed, such that the center 112_3 or 112_4 of the visual field training content 111_3 corresponds to the identified point of view 210_1 or 210_2. By executing the instructions or program code of the visual field training module 136, the at least one processor 140 may display the visual field training content 111_3 at the determined position. Eccentric viewing training through the visual field training content 111_3 of the present disclosure will be described below with reference to FIG. 13.

In an embodiment, the at least one processor 140 may include, but is not limited to, at least one of a CPU, a microprocessor, a graphics processing unit, an application processor (AP), an application-specific integrated circuit (ASIC), a digital signal processor (DSP), a digital signal processing device (DSPD), a programmable logic device (PLD), a field-programmable gate array (FPGA), or a neural processing unit or a dedicated artificial intelligence processor designed with a hardware structure specialized for training and processing of an artificial intelligence model.

In an embodiment, the communication interface 150 may perform data communication with an external server (not shown), under control of the at least one processor 140. In addition, the communication interface 150 may perform data communication not only with the external server but also with other peripheral electronic devices.

In an embodiment, the communication interface 150 may perform data communication with a server or other peripheral electronic devices by using at least one of data communication schemes including, for example, wired local area network (LAN), wireless LAN, Wi-Fi, Bluetooth, ZigBee, Wi-Fi Direct (WFD), Infrared Data Association (IrDA), Bluetooth Low Energy (BLE), near-field communication (NFC), wireless broadband internet (WiBro), Worldwide Interoperability for Microwave Access(WiMAX), Shared Wireless Access Protocol (SWAP), Wireless Gigabit Alliance (WiGig), and radio-frequency (RF) communication.

In an embodiment, the communication interface 150 may also receive, from a server or other peripheral electronic devices, data including the tangent screen 111_1 or 111_2, or the visual field training content 111_3. The at least one processor 140 may also display, on the display 110, the tangent screen 111_1 or 111_2, or the visual field training content 111_3 obtained based on the received data.

In an embodiment, the communication interface 150 may receive, from a server or other peripheral electronic devices, an external input including information about a position at which to display the at least one point 113_1 or 113_2 on the tangent screen 111_1 or 111_2. The at least one processor 140 may calculate, based on the received external input, a position of the at least one point 113_1 or 113_2 to be displayed on the tangent screen 111_1 or 111_2, and display the at least one point 113_1 or 113_2 at the calculated position.

In an embodiment, the communication interface 150 may also receive, from the user 200, a user input including information indicating that the at least one point 113_1 or 113_2 displayed on the tangent screen 111_1 or 111_2 is perceived abnormally. In an embodiment, the communication interface 150 may also receive, via a server or other peripheral electronic devices, a user input including information indicating that the at least one point 113_1 or 113_2 displayed on the tangent screen 111_1 or 111_2 is perceived abnormally. The at least one processor 140 may measure the size and shape of the scotoma 117 of the user 200, based on the received user input.

However, the present disclosure is not limited thereto, and the electronic device 100 may further include an input/output interface. In an embodiment, an input/output interface 170 may perform an input/output operation for data including the tangent screen 111_1 or 111_2, or the visual field training content 111_3, with an external server or an external electronic device by using at least one of input/output methods including High-Definition Multimedia Interface (HDMI), Digital Visual Interface (DVI), and Universal Serial Bus (USB).

FIG. 3 is a diagram for describing an operation of measuring the size and shape of a scotoma of a user by using a tangent screen and at least one point, according to an embodiment of the present disclosure.

FIG. 3 illustrates an operation of measuring the size and shape of the scotoma 117 of the user 200 (see FIG. 1) viewing a tangent screen 111, by using the tangent screen 111 and the at least one point 113 displayed on the tangent screen 111.

In an embodiment, the tangent screen 111 is a planar perimeter that uses a plane, and is also referred to as a Bjerrum screen. The tangent screen 111 is content for measuring the visual field of the user 200 using the tangent screen 111. In an embodiment, the tangent screen 111 is content for measuring the central visual field of the user 200 and measuring the size and shape of a scotoma of the user 200, when the user 200 using the tangent screen is gazing at a center 112 of the tangent screen 111.

Referring to FIGS. 2 and 3, the at least one processor 140 may display the tangent screen 111 on the display 110, and display the at least one point 113 on the tangent screen 111. In an embodiment, the at least one processor 140 may display the at least one point 113 on the tangent screen 111 such that the position of the at least one point 113 changes in a direction from an outermost peripheral position on the tangent screen 111 toward the center 112 of the tangent screen.

In an embodiment, while displaying the point 113 on the tangent screen 111 such that the position of the point 113 changes in a direction from an outermost peripheral position on the tangent screen 111 toward the center 112 of the tangent screen, the at least one processor 140 may obtain a user input including information indicating that the point 113 is perceived abnormally. In an embodiment, the at least one processor 140 may determine, based on the user input, that the at least one point 113 falls within the scotoma of the user 200.

In an embodiment, the user input may be a signal obtained based on a reaction of the user 200 when the point 113 displayed on the tangent screen 111 begins to be perceived abnormally by the user 200 gazing at the center 112 of the tangent screen. In an embodiment, the HMD device 100 may sense a voice signal of the user 200 indicating that the point 113 is perceived abnormally, and generate a user input based on the sensed voice signal.

In an embodiment, the at least one processor 140 may receive, via the communication interface 150, a user input that has been generated by other peripheral electronic devices that have sensed inputs from the user 200 when the point 113 began to be perceived abnormally, and have generated the user input based on the inputs sensed from the user 200. The present disclosure is not limited thereto, and the HMD device 100 may also obtain a user input by sensing an input from the user 200.

When the user input is obtained, the at least one processor 140 may obtain position information 115 about the point that is displayed on the tangent screen 111 at the moment the user input is obtained.

In an embodiment, the at least one processor 140 may repeat the process of changing the position of the point 113 displayed on the tangent screen 111 in a direction from an outermost peripheral position on the tangent screen 111 toward the center 112 of the tangent screen 111 while obtaining a user input and the position information 115 about the at least one point, while shifting, in a clockwise or counterclockwise direction around the tangent screen 111, the position where the point 113 is displayed on the tangent screen 111. However, the present disclosure is not limited thereto, and the position of the at least one point 113 displayed on the tangent screen 111 may also be changed according to the random pattern 114_1 (see FIG. 8A) or the preset position pattern 114_2 (see FIG. 8B).

In an embodiment, the at least one processor 140 may obtain an isopter contour line 116 based on pieces of position information 115 about a plurality of points obtained while changing the position of the at least one point 113 displayed on the tangent screen 111. In an embodiment, the isopter contour line 116 may be a line connecting points of equivalent vision within the visual field of the user 200 gazing at the center 112 of the tangent screen.

In an embodiment, because the isopter contour line 116 is obtained by connecting a plurality of pieces of position information 115 where the point 113 displayed on the tangent screen 111 is perceived abnormally, the at least one processor 140 may determine the isopter contour line 116 and the area 117 inside the isopter contour line 116 as a visual field defect area of the user 200, and determine this area as the scotoma 117. In an embodiment, the at least one processor 140 may measure the size and shape of the scotoma 117 of the user 200 based on the size and shape of the isopter contour line 116.

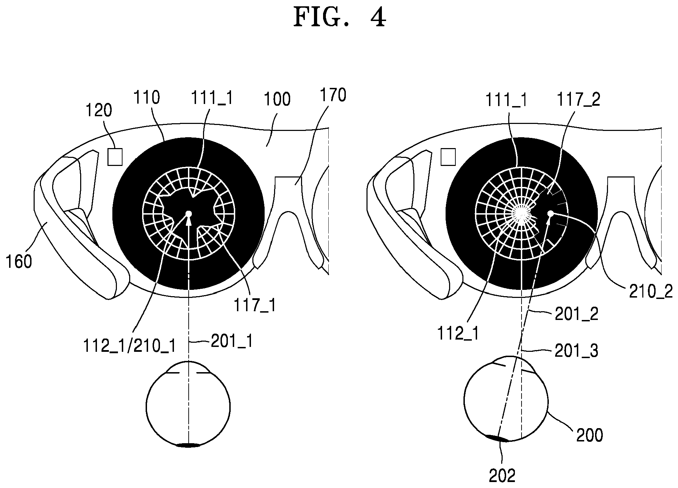

FIG. 4 is a diagram for describing an HMD device and operations of the HMD device, according to an embodiment of the present disclosure. Hereinafter, the same reference numerals are assigned to the same components as those described above with reference to FIGS. 1 and 3, and redundant descriptions thereof will be omitted.

Referring to FIG. 4, the HMD device 100 may include the display 110, the eye-tracking sensor 120, a frame 160, and a nose support 170. In an embodiment, FIG. 4 discloses only components for describing a structure of the HMD device 100, and the components included in the HMD device 100 are not limited to those illustrated in FIG. 4.

In an embodiment, the HMD device 100 may further include a camera for photographing a physical environmental space viewed by the user 200, a sensing unit for sensing an input from the user 200 when the point 113_1 (see FIG. 8A) displayed on the tangent screen 111_1 begins to be perceived abnormally by the user 200, a battery, and the like. In addition, the HMD device 100 may include the components illustrated in FIG. 2.

In an embodiment, the display 110 may be arranged on the frame 160. Although FIG. 4 illustrates the display 110 as having a circular shape, the present disclosure is not limited thereto. The display 110 may have a quadrangular shape or the like.

In an embodiment, the frame 160 may have a shape similar to a frame of a general eyeglass structure. The frame 160 may include a rim surrounding the display 110 and temple portions that extend over ear parts of the user 200 to support the HMD device 100 when the user 200 wears the HMD device 100. In an embodiment, in a case in which the HMD device 100 includes a memory, a processor, a battery, and the like, the memory, the processor, the battery, and the like may be embedded in the temple portions of the frame 160.

In an embodiment, the nose support 170 may be connected to the frame 160. The nose support 170 is a portion that extends over a nose part of the user 200 to support the HMD device 100 when the user 200 wears the HMD device 100. In an embodiment of the present disclosure, the nose support 170 may include a bridge and nose pads. In addition, the bridge and the nose pads may be configured integrally, but are not limited thereto. In an embodiment, the nose support 170 and the frame 160 may also be formed integrally.

In an embodiment, the HMD device 100 may further include a camera connected to the frame 160. The camera may be oriented in a direction opposite to the direction in which the display 110 faces the user, so as to photograph a physical environmental space viewed by the user 200.

In an embodiment, the HMD device 100 may obtain, via the eye-tracking sensor 120, the gaze direction 201_1 of the user 200 wearing the HMD device 100, and the point of view 210_1 where the gaze of the user 200 intersects the display 110.

In an embodiment, the HMD device 100 may determine a position on the display 110 at which to display the tangent screen 111_1 such that the center of the tangent screen 111_1 corresponds to the point of view 210_1. The HMD device 100 may display the tangent screen 111_1 at the determined position. The HMD device 100 may display the at least one point 113_1 (see FIG. 8A) on the tangent screen 111_1, change the position of the displayed at least one point 113_1, and obtain, from the user 200, a user input including information indicating that the at least one point 113_1 is perceived abnormally.

In an embodiment, the HMD device 100 may obtain the size and shape of a scotoma 117_1 of the user, based on the obtained user input.

When macular degeneration occurs in a macula 202 of the user, a scotoma may be located in the center of the visual field of the user, and thus, central vision loss may occur in the user 200. In an embodiment, during the process of obtaining the size and shape of the scotoma 117_1 of the user by displaying the tangent screen 111_1 and the at least one point 113_1 on the HMD device 100, the point of view 210_1 of the user needs to correspond to the center 112_1 of the tangent screen as the user 200 gazes at the center 112_1 of the tangent screen, in order to accurately obtain the size and shape of the scotoma 117_1.

Due to central vision loss, the user 200 may be unable to perceive the center 112_1 of the tangent screen when gazing at the center 112_1 of the tangent screen. Thus, the user 200 may gaze at a peripheral area rather than the center 112_1 of the tangent screen, in order to perceive the position of the center 112_1 of the tangent screen 111_1 or the like by using peripheral vision 201_3. Thus, the point of view 210_2 of the user may not correspond to the center 112_1 of the tangent screen displayed on the display 110.

In this case, the user 200, using the peripheral vision 201_3, may normally perceive the at least one point 113_1, which may appear abnormal in a case in which the user 200 is gazing at the center 112_1 of the tangent screen. In addition, when the user 200 is gazing at the center 112_1 of the tangent screen, the at least one point 113_1, which may appear normal when using the peripheral vision 201_3, may fall within the scotoma 117_2 of the user and thus appear abnormal. Thus, during the process of measuring the size and shape of the scotoma 117_1 or 117_2 of the user by using the tangent screen 111_1 and the at least one point 113_1, when the gaze direction 201_1 or 201_2 of the user changes, the scotoma 117_1 or 117_2 of the user may not be obtained accurately.

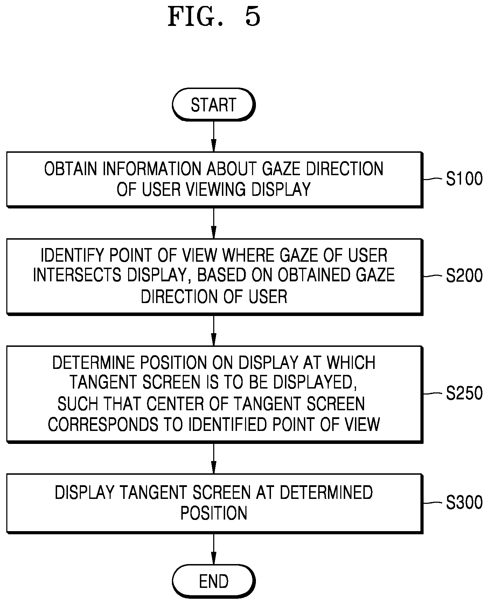

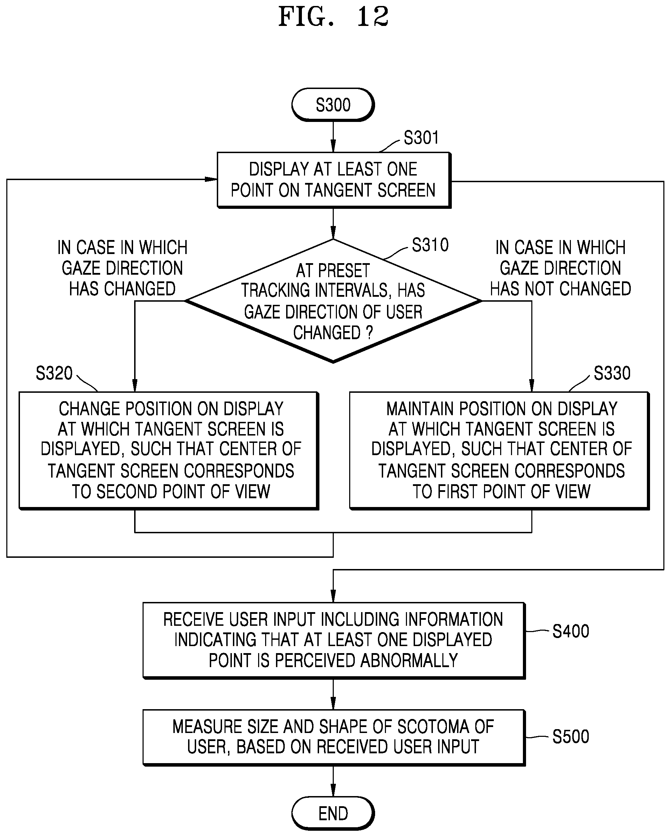

Hereinafter, obtaining the accurate size and shape of the scotoma 117_1 of the user by displaying the tangent screen 111_1 on the display 110 such that the center 112_1 of the tangent screen corresponds to the point of view 210_1 of the user, via the HMD device 100 and the operating method of the HMD device 100 of the present disclosure will be described with reference to FIGS. 5 to 12. In addition, obtaining, during the process of measuring the size and shape of the scotoma 117_1 of the user, the accurate size and shape of the scotoma 117_1 of the user by changing the position of the tangent screen 111_2 displayed on the display 110 such that the center 112_2 (see FIG. 12) of the tangent screen displayed on the display 110 corresponds to the changed point of view 210_2 of the user, when the gaze direction 201_1 or 201_2 of the user changes will be described.

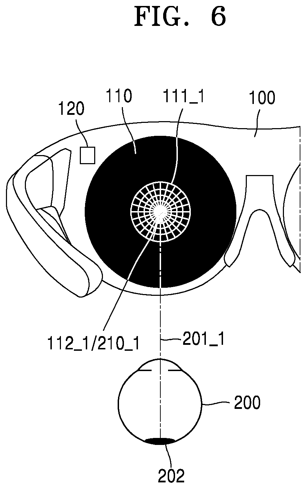

FIG. 5 is a flowchart for describing an operating method of an HMD device according to an embodiment of the present disclosure. FIG. 6 is a diagram for describing displaying a tangent screen on a display such that the center of the tangent screen corresponds to a point of view, according to an embodiment of the present disclosure. Hereinafter, the same reference numerals are assigned to the same components as those described above with reference to FIGS. 1 and 4, and redundant descriptions thereof will be omitted.

Referring to FIGS. 5 and 6, in an embodiment, the operating method of the HMD device 100 may include obtaining information about the gaze direction 201_1 of the user viewing the display 110 (S100). In an embodiment, in the obtaining of the information about the gaze direction 201_1 of the user (S100), the at least one processor 140 (see FIG. 2) may obtain the gaze direction 201_1 of the user, via the eye-tracking sensor 120.

In an embodiment, the operating method of the HMD device 100 may include identifying the point of view 210_1 where the gaze of the user 200 intersects the display 110, based on the obtained gaze direction 201_1 of the user (S200). In an embodiment, in the identifying of the point of view 210_1 (S200), the at least one processor 140 may identify the point of view 210_1 where the gaze of the user 200 intersects the display 110, based on the gaze direction 201_1 of the user obtained via the eye-tracking sensor 120, and a calculated distance between the eye of the user 200 and the display 110.

In an embodiment, the operating method of the HMD device 100 may include determining a position on the display 110 at which the tangent screen 111_1 is to be displayed, such that the center 112_1 of the tangent screen corresponds to the identified point of view 210_1 (S250). In an embodiment, in the determining of the position on the display 110 at which the tangent screen 111_1 is to be displayed (S250), the at least one processor 140 may determine the position of the tangent screen 111_1 based on the identified point of view 210_1, such that the center 112_1 of the tangent screen corresponds to the identified point of view 210_1.

In an embodiment, the operating method of the HMD device 100 may include displaying the tangent screen 111_1 at the determined position (S300). In an embodiment, in the displaying of the tangent screen 111_1 on the display 110 (S300), the at least one processor 140 may display the tangent screen 111_1 at the position determined such that the center 112_1 of the tangent screen corresponds to the identified point of view 210_1. In an embodiment, the at least one processor 140 may provide the tangent screen 111_1 to the user 200 viewing the display 110.

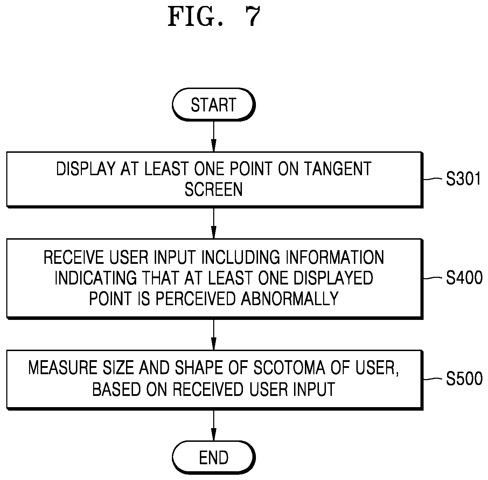

FIG. 7 is a flowchart for describing a method of measuring the size and shape of a scotoma of a user by displaying a tangent screen and at least one point, according to an embodiment of the present disclosure. FIG. 8A is a diagram for describing changing the position of at least one point according to a random pattern, according to an embodiment of the present disclosure. FIG. 8B is a diagram for describing changing the position of at least one point according to a preset position pattern, according to an embodiment of the present disclosure. FIG. 9 is a diagram for describing obtaining a notification signal for at least one point and measuring the size and shape of a scotoma of a user based on the obtained notification signal, according to an embodiment of the present disclosure. Hereinafter, the same reference numerals are assigned to the same components and operations as those described above with reference to FIGS. 1, 3, and 5, and redundant descriptions thereof will be omitted.

Referring to FIGS. 7 and 8A, in an embodiment, the operating method of the HMD device 100 may include, after the displaying of the tangent screen 111_1 on the display 110 (S300), displaying the at least one point 113_1 on the tangent screen 111_1 (S301). In an embodiment, in the displaying of the at least one point 113_1 on the tangent screen 111_1 (S301), the at least one processor 140 may display the at least one point 113_1 on the tangent screen 111_1 to overlap with the tangent screen 111_1 displayed on the display 110. In an embodiment, the at least one processor 140 may display a single point on the tangent screen 111_1 to overlap with the tangent screen 111_1, and shift the position of the displayed single point. In an embodiment, the at least one processor 140 may display a plurality of points on the tangent screen 111_1 during a plurality of frames. Here, the position of a point displayed on the tangent screen 111_1 in each frame may be changed.

FIG. 8A illustrates that the at least one processor 140 displays the at least one point 113_1 on the tangent screen 111_1 while changing the position of the at least one point 113_1 to correspond to the random pattern 114_1.

In an embodiment, in the displaying of the at least one point 113_1 on the tangent screen 111_1 (S301), the position of the at least one point 113_1 may be randomly changed. In an embodiment, in the displaying of the at least one point 113_1 on the tangent screen 111_1 (S301), the at least one processor 140 may display the at least one point 113_1 on the tangent screen 111_1 while randomly changing the position of the at least one point 113_1. In an embodiment, the at least one processor 140 may display a plurality of points on the tangent screen 111_1 during a plurality of frames. Here, the position of a point displayed on the tangent screen 111_1 in each frame may be randomly changed.

In an embodiment, when the at least one point 113_1 is displayed on the tangent screen 111_1 while randomly changing the position of the at least one point 113_1, the user 200 cannot predict the position of the point to be displayed in the next frame, based on the position of the point displayed in the current frame. Thus, it is possible to prevent reception of an incorrect user input generated to include information indicating that a point that does not actually fall within the scotoma 117 (see FIG. 9) is perceived abnormally because the user 200 has predicted the position of the point to be displayed in the next frame.

In an embodiment, in the displaying of the at least one point 113_1 on the tangent screen 111_1 (S301), the at least one point 113_1 may be displayed such that its shape corresponds to any one of a plurality of preset shapes. In an embodiment, in the displaying of the at least one point 113_1 on the tangent screen 111_1 (S301), the at least one processor 140 may display the at least one point 113_1 such that its shape corresponds to any one of a plurality of preset shapes.

In an embodiment, the plurality of preset shapes may include Arabic numerals, arrows, special characters, and the like. The at least one processor 140 may display a plurality of points on the tangent screen 111_1 during a plurality of frames. Here, the at least one processor 140 may display the point displayed on the tangent screen 111_1 in each frame such that the shape of the point corresponds to any one shape selected from among the plurality of preset shapes. In an embodiment, the shape of the point displayed on the tangent screen 111_1 in each frame may correspond to any one shape randomly selected from among the plurality of preset shapes.

In an embodiment, FIG. 8A illustrates four points 113_1 displayed in four frames, respectively. Here, the position of the point in each of the four frames may have been randomly changed. The shapes of the points in the four frames are illustrated as '3', '2', '8', and '1', respectively. In an embodiment, the shapes '3', '2', '8', and '1' may be shapes randomly selected from a plurality of preset shapes including Arabic numerals.

In an embodiment, when the shape of the at least one point 113_1 is randomly changed every frame and then displayed on the tangent screen 111_1, the user 200 cannot predict the shape of the point to be displayed in the next frame based on the shape of the point displayed in the current frame. Thus, it is possible to prevent an inaccurate size and shape of the scotoma 117 from being obtained due to receiving an incorrect user input generated because the user 200 has predicted the shape of the point to be displayed in the next frame and thus indicated a normal perception response for the next point, which actually falls within the scotoma 117 and thus is perceived abnormally.

In an embodiment, "3", "2", "8", and "1" illustrated in FIG. 8A are not related to the order of the at least one point 113_1 displayed on the tangent screen 111_1, and the at least one point 113_1 displayed sequentially at random positions may have a shape randomly selected from among the plurality of preset shapes. In an embodiment, the at least one point 113_3 may have a shape randomly selected from among shapes including not only Arabic numerals but also figures, arrows, and the like.

FIG. 8B illustrates that the at least one processor 140 displays the at least one point 113_2 on the tangent screen 111_1 while changing the position of the at least one point 113_2 to correspond to the preset position pattern 114_2.

Referring to FIGS. 7 and 8B, in an embodiment, in the displaying of the at least one point 113_2 on the tangent screen 111_1 (S301), the position of the at least one point 113_2 may be changed to correspond to the preset position pattern 114_2. In an embodiment, in the displaying of the at least one point 113_2 on the tangent screen 111_1 (S301), the at least one processor 140 may display the at least one point 113_2 on the tangent screen 111_1 while changing the position of the at least one point 113_2 according to the preset position pattern 114_2. In an embodiment, while the at least one processor 140 displays a plurality of points on the tangent screen 111_1 during a plurality of frames, the position of the point displayed on the tangent screen 111_1 in each frame may be changed according to the preset position pattern 114_2.

Although FIG. 8B illustrates that the preset position pattern 114_2 is set in a direction from an outermost peripheral position of the tangent screen 111_1 toward the center 112_1 of the tangent screen 111_1, along the outermost periphery of the tangent screen 111_1 clockwise, the present disclosure is not limited thereto. The position pattern may be preset to extend from the center 112_1 of the tangent screen 111_1 toward the outermost periphery, or may be preset counterclockwise along the outermost periphery of the tangent screen 111_1, and is not limited to any particular pattern.

In an embodiment, the operating method of the HMD device 100 may further include receiving an external input including information about a position on the tangent screen 111_1 at which the at least one point 113_2 is to be displayed. In an embodiment, the external input may be received from a server or other peripheral electronic devices. In an embodiment, the external input may be an input for providing information about the position of the at least one point 113_2 provided to the user 200 via the HMD device 100.

In an embodiment, the operating method of the HMD device 100 may further include calculating a position of the at least one point 113_2 to be displayed on the tangent screen 111_1, based on the received external input. In an embodiment, in the displaying of the at least one point 113_2 on the tangent screen 111_1 (S301), the at least one point 113_2 may be displayed at the calculated position.

In an embodiment, the at least one processor 140 may receive an external input including information about a position on the tangent screen 111_1 at which the at least one point 113_2 is to be displayed. The at least one processor 140 may calculate, based on the received external input, a position of the at least one point 113_1 to be displayed on the tangent screen 111_1, and display the at least one point 113_1 at the calculated position.

In an embodiment, when the at least one point 113_2 displayed on the tangent screen 111_1 is displayed at a position calculated via an arbitrary external input, rather than according to the random pattern 114_1 or the preset position pattern 114_2, a visual field test for a desired point may be performed again based on a state or reaction of the user 200 or accumulated test results, so as to measure the accurate size and shape of the scotoma 117.

Referring to FIGS. 7, 8A, 8B, and 9, in an embodiment, the operating method of the HMD device 100 may include receiving, from the user, a user input including information indicating that the at least one point 113_1 or 113_2 displayed on the tangent screen 111_1 is perceived abnormally (S400). In an embodiment, in the receiving of a user input (S400), the at least one processor 140 may obtain a user input including information indicating that the at least one point 113_1 or 113_2 is perceived abnormally, from the user 200 viewing the tangent screen 111_1 and the at least one point 113_1 or 113_2.

In an embodiment, the operating method of the HMD device 100 may include measuring the size and shape of the scotoma 117 of the user, based on the received user input (S500). In an embodiment, in the measuring of the size and shape of the scotoma 117 of the user (S500), the at least one processor 140 may measure, based on the user input, the size and shape of the scotoma 117 of the user viewing the tangent screen 111_1.

In an embodiment, the measuring of the size and shape of the scotoma 117 of the user (S500) may include obtaining, based on the received user input, the position information 115 about a point that is perceived abnormally by the user 200, among the at least one point 113_1 or 113_2 displayed on the tangent screen 111_1. In an embodiment, based on the received user input, the at least one processor 140 may obtain the position information 115 about a point that is perceived abnormally by the user 200, among a plurality of points displayed on the tangent screen 111_1 during a plurality of frames. In an embodiment, when displaying the plurality of points on the tangent screen 111_1 while changing their positions according to the preset position pattern 114_2 during the plurality of frames, the position information 115 may be position information 115 about a point displayed on the tangent screen 111_1 that the user 200 begins to perceive abnormally. In an embodiment, the position information 115 may be obtained corresponding to each of a plurality of directions to be measured via the tangent screen 111_1, relative to the center 112_1 of the tangent screen.

In an embodiment, the measuring of the size and shape of the scotoma 117 of the user (S500) may include obtaining the isopter contour line 116 based on a plurality of obtained pieces of position information 115. In an embodiment, the at least one processor 140 may obtain the isopter contour line 116 based on pieces of position information 115 about a plurality of points obtained while changing the position of the at least one point 113 displayed on the tangent screen 111.

In an embodiment, the measuring of the size and shape of the scotoma 117 of the user (S500) may include measuring the size and shape of the scotoma 117 of the user, based on the obtained isopter contour line 116. In an embodiment, the at least one processor 140 may determine, as the scotoma 117, the obtained isopter contour line 116 and the area 117 inside the isopter contour line 116, and measure the size and shape of the scotoma 117.

According to the HMD device 100 and the operating method of the HMD device 100 of the present disclosure, the gaze direction 201_1 (see FIG. 6) of the user 200 wearing the HMD device 100 is obtained, the point of view 210_1 of the user is identified based on the obtained gaze direction 201_1, the position of the tangent screen 111_1 is determined such that the center 112_1 of the tangent screen corresponds to the identified point of view 210_1, and the tangent screen 111_1 is displayed at the determined position. Thereafter, the size and shape of the scotoma 117 of the user is measured based on the position information 115 obtained by displaying the at least one point 113_1 or 113_2 on the tangent screen 111_1.

According to the HMD device 100 and the operating method of the HMD device 100 of the present disclosure, because the size and shape of the scotoma 117 of the user is measured in a state in which the point of view 210_1 of the user corresponds to the center 112_1 of the tangent screen, the accuracy and reliability of the measurement of the size and shape of the scotoma 117 may be increased.

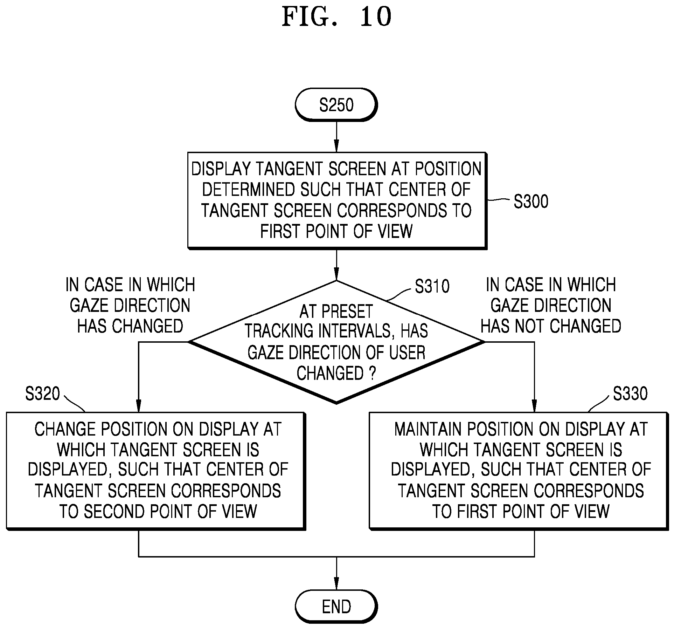

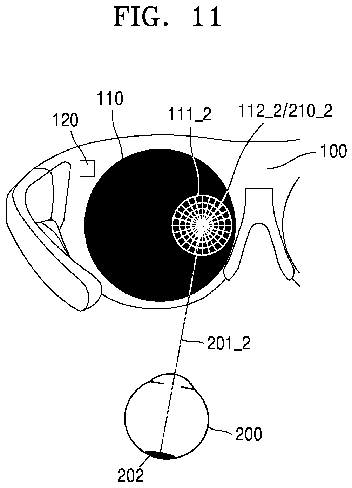

FIG. 10 is a flowchart for describing a method of identifying, at preset tracking intervals, whether a gaze direction of a user has changed, and changing the position of a tangent screen, according to an embodiment of the present disclosure. FIG. 11 is a diagram for describing changing, when a gaze direction of a user has changed, the position of a tangent screen such that the center of the tangent screen corresponds to a changed point of view, according to an embodiment of the present disclosure. Hereinafter, the same reference numerals are assigned to the same components and operations as those described above with reference to FIGS. 5 to 9, and redundant descriptions thereof will be omitted.

Referring to FIGS. 6, 10, and 11, in an embodiment, when the point of view 210_1 of the user obtained in the identifying of the point of view 210_1 where the gaze of the user 200 intersects the display 110 (S200) is referred to as a first point of view 210_1, in the displaying of the tangent screen 111_1 on the display 110 (S300), the tangent screen 111_1 may be displayed on the display 110 such that the center 112_1 of the tangent screen corresponds to the first point of view 210_1.

In an embodiment, the operating method of the HMD device 100 may further include, after the displaying of the tangent screen 111_1 at the position determined such that the center 112_1 of the tangent screen corresponds to the first point of view 210_1 (S300), identifying, at preset tracking intervals, whether the gaze direction 201_1 of the user has changed (S310). In an embodiment, after displaying the tangent screen 111_1 on the display 110 such that the center 112_1 of the tangent screen corresponds to the first point of view 210_1, the at least one processor 140 may determine whether the gaze direction 201_1 of the user, which is obtained via the eye-tracking sensor 120 at preset tracking intervals, has changed.

In an embodiment, according to a preset determination criterion, the at least one processor 140 may determine, when a change in the obtained gaze direction 201_1 of the user is greater than the preset determination criterion, that the obtained gaze direction 201_1 of the user has changed. In an embodiment, the at least one processor 140 may determine, when a change in the obtained gaze direction 201_1 of the user is less than or equal to the preset determination criterion, that the obtained gaze direction 201_1 of the user has not changed. Here, the preset determination criterion may be set based on the degree of change in the gaze direction of the user, the position of the iris of the user, or the like.

In an embodiment, the preset tracking interval may be an interval set for performing an operation of obtaining information about the gaze direction 201_1 of the user via the eye-tracking sensor 120. For example, in a case in which the preset tracking interval is 3 seconds, the at least one processor 140 may obtain information about the gaze direction 201_1 of the user via the eye-tracking sensor 120 every 3 seconds, and determine whether the previously obtained gaze direction 201_1 of the user has changed. As the preset tracking interval decreases, an interval during which the size and shape of the scotoma 117 of the user is measured in a state in which the point of view 210_1 or 210_2 of the user and the center 112_1 or 112_2 of the tangent screen do not coincide when the gaze direction 201_1 or 201_2 of the user has changed may be decreased, and thus, the accuracy and reliability of the operation of measuring the size and shape of the scotoma 117 of the user may be increased.

In an embodiment, the operating method of the HMD device 100 may include, when it is determined, in the determining of whether the gaze direction 201_1 of the user has changed (S310), that the gaze direction 201_2 of the user has changed, identifying a second point of view 210_2, where the changed gaze of the user intersects the display 110, based on the changed gaze direction 201_2 of the user.

In an embodiment, the at least one processor 140 may identify the second point of view 210_2 where the changed gaze of the user intersects the display 110, based on the changed gaze direction 201_2 of the user and the distance between the display 110 and the iris of the user 200.

In an embodiment, the operating method of the HMD device 100 may include changing the position on the display 110 at which the tangent screen 111_2 is displayed, such that the center 112_2 of the tangent screen corresponds to the identified second point of view 210_2 (S320). In an embodiment, the at least one processor 140 may change the position on the display 110 at which the tangent screen 111_2 is displayed, such that the center 112_2 of the tangent screen corresponds to the identified second point of view 210_2.

In an embodiment, the operating method of the HMD device 100 includes, when it is determined, in the determining of whether the gaze direction 201_1 of the user has changed (S310), that the gaze direction 201_1 of the user has not changed, maintaining the position on the display 110 at which the tangent screen 111_1 is displayed, such that the center 112_1 of the tangent screen corresponds to the first point of view 210_1 (S330).

In an embodiment, when it is determined that the gaze direction 201_1 of the user has not changed, the at least one processor may not change the position of the tangent screen 111_1 displayed on the display 110 such that the center 112_1 of the tangent screen corresponds to the first point of view 210_1.

FIG. 12 is a flowchart for describing a method of, when a gaze direction of a user has changed, measuring the size and shape of a scotoma of the user while changing the position of a tangent screen, according to an embodiment of the present disclosure. Hereinafter, the same reference numerals are assigned to the same operations as those described above with reference to FIG. 10, and redundant descriptions thereof will be omitted.

Referring to FIGS. 6, 8A, 9, 11, and 12, in an embodiment, the operating method of the HMD device 100 may include, after the displaying of the tangent screen 111_1 on the display 110 (S300), displaying the at least one point 113_1 on the tangent screen 111_1 (S301).

In an embodiment, the operating method of the HMD device 100 may include, after the displaying of the at least one point 113_1 on the tangent screen 111_1 (S301), determining, at preset tracking intervals, whether the gaze direction 201_1 of the user has changed (S310).

In an embodiment, the operating method of the HMD device 100 may include, when it is determined, in the determining of whether the gaze direction 201_1 of the user has changed (S310), that the gaze direction 201_2 of the user has changed, changing the position on the display 110 at which the tangent screen 111_2 is displayed, such that the center 112_2 of the tangent screen corresponds to the second point of view 210_2 (S320).

In an embodiment, after the changing of the position on the display 110 at which the tangent screen 111_2 is displayed such that the center 112_2 of the tangent screen corresponds to the second point of view 210_2 (S320), in the displaying of the at least one point 113_2 (S301), the at least one point 113_2 may be displayed on the tangent screen 111_2 whose position has been changed. In an embodiment, the at least one processor 140 may change the position of the tangent screen 111_2 such that the center 112_2 of the tangent screen corresponds to the second point of view 210_2, then display the tangent screen 111_2 on the display 110, and then display the at least one point 113_2 on the tangent screen 111_2 whose position has been changed.

In an embodiment, the operating method of the HMD device 100 may include, when it is determined, in the determining of whether the gaze direction 201_1 of the user has changed (S310), that the gaze direction 201_1 of the user has not changed, maintaining the position on the display 110 at which the tangent screen 111_1 is displayed, such that the center 112_1 of the tangent screen corresponds to the first point of view 210_1 (S330).

In an embodiment, after the maintaining of the position on the display 110 at which the tangent screen 111_1 is displayed such that the center 112_1 of the tangent screen corresponds to the first point of view 210_1 (S330), in the displaying of the at least one point 113_1 (S301), an operation of displaying the at least one point 113_1 on the tangent screen 111_1 displayed on the display 110, such that the center 112_1 of the tangent screen corresponds to the first point of view 210_1 may be repeated. In an embodiment, the at least one processor 140 may display the at least one point 113_2 on the tangent screen 111_1 displayed on the display 110, such that the center 112_1 of the tangent screen corresponds to the first point of view 210_1.

In an embodiment, the operating method of the HMD device 100 may include receiving, from the user 200, a user input including information indicating that at least one point 113_1 or 113_2 displayed on the tangent screen 111_1 is perceived abnormally (S400).

In an embodiment, the operating method of the HMD device 100 may repeat the determining of whether the gaze direction of the user has changed (S310), and according to a result of the determining of whether the gaze direction of the user has changed (S310), either the displaying on the display 110 such that the tangent screen 112_2 corresponds to the second point of view 210_2 (S320) or the displaying on the display 110 such that the tangent screen 112_1 corresponds to the first point of view 210_1 (S330), and then receive a user input including information indicating that the at least one point 113_1 or 113_2 displayed on the tangent screen 111_1 is perceived abnormally.

Referring to FIGS. 5, 7, and 12, in an embodiment, upon receiving a user input including information indicating that the at least one point 113_1 or 113_2 displayed on the tangent screen 111_1 is perceived abnormally, the operating method of the HMD device 100 may repeat, until a plurality of pieces of position information 115 necessary for measuring the size and shape of the scotoma 117 of the user are obtained, the identifying of the first point of view 210_1 (S200), the displaying of the tangent screen 111_1 at the position determined such that the center 112_1 of the tangent screen corresponds to the identified first point of view 210_1 (S300), the displaying of the at least one point 113_1 on the tangent screen 111_1 while changing its position (S301), the determining of whether the gaze direction 201_2 of the user has changed (S310), the changing, when the gaze direction 201_2 of the user has changed, of the position of the tangent screen 111_2 such that the center 112_2 of the tangent screen corresponds to the changed second point of view 210_2 (S320), and the displaying of the at least one point 113_2 on the changed tangent screen 111_2 while changing the position of the at least one point 113_2 (S301).

In an embodiment, the at least one processor 140 may repeat, until the size and shape of the scotoma 117 of the user is measured, identifying the first point of view 210_1, displaying the tangent screen 111_1 such that the center 112_1 of the tangent screen corresponds to the identified first point of view 210_1, displaying the at least one point 113_1 on the tangent screen 111_1 while changing the position of the at least one point 113_1 and receiving a user input, and, when the gaze direction 201_2 of the user has changed, before measuring the size and shape of the scotoma 117, identifying the changed second point of view 210_2, changing the position of the tangent screen 111_2 such that the center 112_2 of the tangent screen corresponds to the identified second point of view 210_2, and displaying the at least one point 113_1 on the changed tangent screen 111_2 while changing the position of the at least one point 113_1 and obtaining a notification signal.

In an embodiment, the operating method of the HMD device 100 may include, upon receiving a user input including information indicating that the at least one point 113_1 or 113_2 displayed on the tangent screen 111_1 is perceived abnormally, measuring the size and shape of the scotoma of the user (S500). In detail, in the measuring of the size and shape of the scotoma of the user (S500), the at least one processor 140 may measure the size and shape of the scotoma of the user, based on the plurality of pieces of position information 115 obtained through the above-described processes.

FIG. 13 is a diagram for describing displaying visual field training content on a display such that the center of the visual field training content corresponds to a point of view, according to an embodiment of the present disclosure. Hereinafter, the same reference numerals are assigned to the same components as those described above with reference to FIGS. 6, 9, and 11, and redundant descriptions thereof will be omitted.

In an embodiment, the operating method of the HMD device 100 may further include, after the measuring of the size and shape of the scotoma 117 of the user (S500), displaying, on the display 110, the visual field training content 111_3 or 111_4 for training the visual field of the user 200, based on the measured size and shape of the scotoma 117 of the user.

In an embodiment, the visual field training content 111_3 or 111_4 is content for performing eccentric viewing training such that the user 200, who has the scotoma 117 in the center of the visual field due to a visual field impairment such as macular degeneration, may perceive objects through peripheral vision. In an embodiment, the visual field training content 111_3 or 111_4 may be spaced apart from the point of view 210_1 or 210_2 of the user by a certain interval, and thus may not fall within the scotoma 117. According to the HMD device 100 and the operating method of the HMD device 100 of the present disclosure, the user 200 may perform eccentric viewing training to recognize, by using peripheral vision, the visual field training content 111_3 or 111_4 that is spaced apart from the point of view 210_1 or 210_2 of the user by a certain interval and thus does not fall within the scotoma 117. Although FIG. 13 illustrates that the scotoma 117 of the user is also displayed on the display 110, this is for explaining that the visual field training content 111_3 or 111_4 does not fall within the scotoma 117 of the user, and the scotoma 117 of the user may not actually be displayed on the display 110.

In an embodiment, the displaying of the visual field training content 111_3 on the display 110 may include determining a position on the display 110 at which the visual field training content 111_3 or 111_4 is to be displayed, such that the center 112_3 or 112_4 of the visual field training content 111_3 corresponds to the identified point of view 210_1 or 210_2. In an embodiment, the center of the visual field training content 111_3 or 111_4 may be a reference point for maintaining a constant interval between the visual field training content 111_3 perceived by the user 200 and the point of view 210_1 or 210_2 of the user.

In an embodiment, the displaying of the visual field training content 111_3 on the display 110 may include displaying the visual field training content 111_3 at the determined position.

In an embodiment, the displaying of the visual field training content 111_3 on the display 110 may include, when the gaze direction 201_2 of the user has changed during the eccentric viewing training of the user 200, changing the position of the visual field training content 111_3 displayed on the display 110 such that the center 112_3 of the visual field training content 111_3 corresponds to the identified second point of view 210_2.

In an embodiment, the at least one processor 140 may display, on the display 110, the visual field training content 111_3 for training the visual field of the user 200, based on the measured size and shape of the scotoma 117 of the user.

In an embodiment, the at least one processor 140 may determine the position on the display 110 at which the visual field training content 111_3 is to be displayed, such that the center 112_3 or 112_4 of the visual field training content corresponds to the identified point of view 210_1 or 210_2 of the user, and display the visual field training content 111_3 at the determined position.

According to the HMD device 100 and the operating method of the HMD device 100 of the present disclosure, even when the gaze direction 201_1 or 201_2 of the user changes while the user 200 is performing eccentric viewing training, the visual field training content 111_3 perceived by the user 200 using peripheral vision may be displayed on the display 110 while maintaining a constant interval from the point of view 210_1 or 210_2 of the user. Thus, the accuracy and effectiveness of the eccentric viewing training may be increased.

To solve the above-described technical issues, an embodiment provides an HMD device 100 including a display 110 configured to display a tangent screen 111_1 or 111_2 for measuring a size and a shape of a scotoma 117, an eye-tracking sensor 120 configured to obtain information about a gaze direction 201_1 or 201_2 of a user 200, a memory 130 storing at least one instruction, and at least one processor 140 configured to execute the at least one instruction stored in the memory 130. In an embodiment, the at least one processor 140 may identify a point of view 210_1 or 210_2 where a gaze of the user 200 intersects the display 110, based on the gaze direction 201_1 or 201_2 of the user obtained via the eye-tracking sensor 120. The at least one processor 140 may determine a position on the display 110 at which the tangent screen 111_1 or 111_2 is to be displayed, such that a center 112_1 or 112_2 of the tangent screen corresponds to the identified point of view 210_1 or 210_2. The at least one processor 140 may display the tangent screen 111_1 or 111_2 at the determined position.

In an embodiment, a point of view where the gaze of the user 200 intersects the display 110 may be referred to as a first point of view 210_1. When the obtained gaze direction 201_1 of the user 200 is changed, the at least one processor 140 may identify, based on the changed gaze direction 201_2 of the user 200, a second point of view 210_2 where the changed gaze of the user intersects the display 110. The at least one processor 140 may change the position on the display 110 at which the tangent screen 111_2 is displayed, such that the center 112_2 of the tangent screen corresponds to the identified second point of view.

In an embodiment, after displaying the tangent screen 111_1 on the display 110 such that the center 112_1 of the tangent screen corresponds to the first point of view 210_1, the at least one processor 140 may determine whether the gaze direction 201_1 of the user 200, which is obtained via the eye-tracking sensor 120 at preset tracking intervals, has changed.

In an embodiment, the at least one processor 140 may display at least one point 113_1 or 113_2 on the tangent screen 111_1 or 111_2. The at least one processor 140 may receive, from the user, a user input including information indicating that the displayed at least one point 113_1 or 113_2 is perceived abnormally. The at least one processor 140 may measure the size and shape of the scotoma 117 of the user 200, based on the received user input.

In an embodiment, the at least one processor 140 may change a position of the at least one point 113_1 or 113_2 displayed on the tangent screen 111_1 or 111_2, according to a random pattern 114_1.

In an embodiment, the at least one processor 140 may change a position of the at least one point 113_1 or 113_2 displayed on the tangent screen 111_1 or 111_2, according to a preset position pattern 114_2.

In an embodiment, the at least one processor 140 may receive an external input including information about a position at which the at least one point 113_1 or 113_2 is to be displayed on the tangent screen 111_1 or 111_2. The at least one processor 140 may calculate a position of the at least one point 113_1 or 113_2 to be displayed on the tangent screen 111_1 or 111_2, based on the received external input. The at least one processor 140 may display the at least one point 113_1 or 113_2 at the calculated position.

In an embodiment, the at least one processor 140 may display a shape of the at least one point 113_1 or 113_2 such that the shape corresponds to any one of a plurality of preset shapes.

In an embodiment, the at least one processor 140 may measure the size and the shape of the scotoma 117 of the user 200 via the tangent screen 111_1 or 111_2. The at least one processor 140 may display, on the display 110, visual field training content 111_3 for training a visual field of the user 200, based on the measured size and shape of the scotoma 117 of the user 200.

In an embodiment, the at least one processor 140 may determine a position on the display 110 at which the visual field training content 111_3 is to be displayed, such that a center 112_3 or 112_4 of the visual field training content corresponds to the identified point of view 210_1 or 210_2. The at least one processor 140 may display the visual field training content 111_3 at the determined position.

To solve the above-described technical issues, another embodiment of the present disclosure provides an operating method of an HMD device 100 including a display 110. The operating method of the HMD device 100 may include obtaining information about a gaze direction 201_1 or 201_2 of a user viewing the display 110 (S100). The operating method of the HMD device 100 may include identifying, based on the obtained gaze direction 201_1 or 201_2 of the user 200, a point of view 210_1 or 210_2 where a gaze of the user 200 intersects the display 110 (S200). The operating method of the HMD device 100 may include determining a position on the display 110 at which a tangent screen 111_1 or 111_2 is to be displayed, such that a center 112_1 or 112_2 of the tangent screen for measuring a size and a shape of a scotoma 117 of the user corresponds to the identified point of view 210_1 or 210_2 (S250). The operating method of the HMD device 100 may include displaying the tangent screen 111_1 or 111_2 at the determined position (S300).

In an embodiment, the point of view identified in the identifying of the point of view where the gaze of the user 200 intersects the display 110 (S200) may be referred to as a first point of view 210_1. The operating method of the HMD device 100 may include, after the displaying of the tangent screen 111_1 at the determined position such that the center 112_1 of the tangent screen corresponds to the first point of view 210_1 (S300), determining whether the gaze direction 201_2 of the user has changed (S310). The operating method of the HMD device 100 may include identifying, based on the changed gaze direction 201_2 of the user, a second point of view 210_2 where the changed gaze of the user 200 intersects the display 110. The operating method of the HMD device 100 may further include changing the position on the display 110 at which the tangent screen 111_2 is displayed, such that the center 112_2 of the tangent screen corresponds to the identified second point of view 210_2 (S320).

In an embodiment, the determining of whether the gaze direction 201_2 of the user has changed (S310) may further include determining, at preset tracking intervals, whether the gaze direction 201_1 or 201_2 of the user 200 has changed (S310).

In an embodiment, the operating method of the HMD device 100 may further include displaying at least one point 113_1 or 113_2 on the tangent screen 111_1 or 111_2 (S301). The operating method of the HMD device 100 may further include receiving, from the user 200, a user input including information indicating that the displayed at least one point 113_1 or 113_2 is perceived abnormally (S400). The operating method of the HMD device 100 may further include measuring the size and the shape of the scotoma 117 of the user 200, based on the received user input (S500).

In an embodiment, in the displaying of the at least one point 113_1 or 113_2 (S301) in the operating method of the HMD device 100, a position of the at least one point 113_1 or 113_2 displayed on the tangent screen 111_1 or 111_2 may be randomly changed.

In an embodiment, in the displaying of the at least one point 113_1 or 113_2 (S301) in the operating method of the HMD device 100, a position of the at least one point 113_1 or 113_2 displayed on the tangent screen 111_1 or 111_2 may be changed to correspond to a preset position pattern 114_4.

In an embodiment, the operating method of the HMD device 100 may further include receiving an external input including information about a position on the tangent screen 111_1 or 111_2 at which the at least one point 113_2 or 113_2 is to be displayed. The operating method of the HMD device 100 may further include calculating a position of the at least one point 113_1 or 113_2 to be displayed on the tangent screen 111_1 or 111_2, based on the received external input. In the displaying of the at least one point 113_1 or 113_2 (S301) in the operating method of the HMD device 100, the at least one point 113_1 or 113_2 may be displayed at the calculated position.

In an embodiment, in the displaying of the at least one point 113_1 or 113_2 (S301) in the operating method of the HMD device 100, a shape of the at least one point 113_1 or 113_2 may be displayed such that the shape corresponds to any one of a plurality of preset shapes.

In an embodiment, the operating method of the HMD device 100 may further include, after the measuring of the size and the shape of the scotoma 117_1 or 117_2 of the user 200 (S500), displaying, on the display 110, visual field training content 111_3 for training a visual field of the user 200, based on the measured size and shape of the scotoma 117 of the user 200. In the displaying of the visual field training content 111_3 on the display 110, the operating method of the HMD device 100 may include determining a position on the display 110 at which the visual field training content 111_3 is to be displayed, such that a center 112_3 or 112_4 of the visual field training content corresponds to the identified point of view 210_1 or 210_2. In the displaying of the visual field training content 111_3 on the display 110, the operating method of the HMD device 100 may include displaying the visual field training content 111_3 at the determined position.

An embodiment may provide a computer-readable recording medium having recorded thereon a program for causing a computer to execute the operating method of the electronic device 100 according to at least one of embodiments.

A program executable by the HMD device 100 described herein may be implemented as a hardware component, a software component, and/or a combination of hardware components and software components. The program is executable by any system capable of executing computer-readable instructions.

The software may include a computer program, code, instructions, or a combination of one or more thereof, and may configure the processor to operate as desired or may independently or collectively instruct the processor.

The software may be implemented as a computer program that includes instructions stored in computer-readable storage media. The computer-readable storage media may include, for example, magnetic storage media (e.g., ROM, RAM, floppy disks, or hard disks) and optical storage media (e.g., a compact disc ROM (CD-ROM) or a digital versatile disc (DVD)). The computer-readable recording medium may be distributed in computer systems connected via a network and may store and execute computer-readable code in a distributed manner. The recording medium may be computer-readable, may be stored in a memory, and may be executed by a processor.

The computer-readable storage medium may be provided in the form of a non-transitory storage medium. Here, the term 'non-transitory storage medium' refers to a tangible device and does not include a signal (e.g., an electromagnetic wave), and the term 'non-transitory storage medium' does not distinguish between a case where data is stored in a storage medium semi-permanently and a case where data is stored temporarily. For example, the 'non-transitory storage medium' may include a buffer in which data is temporarily stored.

In addition, a program according to embodiments disclosed herein may be provided in a computer program product. The computer program product may be traded as commodities between sellers and buyers.

The computer program product may include a software program and a computer-readable recording medium storing the software program. For example, the computer program product may include a product (e.g., a downloadable application) in the form of a software program electronically distributed through a manufacturer of the HMD device 100 or an electronic market (e.g., Samsung Galaxy Store). For electronic distribution, at least part of the software program may be stored in a storage medium or temporarily generated. In this case, the storage medium may be a storage medium of a server of the manufacturer of the HMD device 100, a server of the electronic market, or a relay server that temporarily stores the software program.

Although the embodiments have been described with the limited embodiments and the drawings, various modifications and changes may be made by those of skill in the art from the above description. For example, suitable results may be obtained even when the described techniques are performed in a different order, or when components in a described electronic device, architecture, device, or circuit are coupled or combined in a different manner, or replaced or supplemented by other components or their equivalents.

## Claims

1. A head-mounted display device (100) comprising:
a display (110) configured to display a tangent screen (111_1 or 111_2) for measuring a size and a shape of a scotoma (117);
an eye-tracking sensor (120) configured to obtain information about a gaze direction (201_1 or 201_2) of a user (200);
a memory (130) storing at least one instruction; and
at least one processor (140) configured to execute the at least one instruction stored in the memory (130),
wherein the at least one processor (140) is further configured to identify a point of view (210_1 or 210_2) where a gaze of the user (200) intersects the display (110), based on the gaze direction (201_1 or 201_2) of the user obtained via the eye-tracking sensor (120), determine a position on the display (110) at which the tangent screen (111_1 or 111_2) is to be displayed, such that a center (112_1 or 112_2) of the tangent screen corresponds to the identified point of view (210_1 or 210_2), and display the tangent screen (111_1 or 111_2) at the determined position.

2. The head-mounted display device (100) of claim 1, wherein a point of view where the gaze of the user (200) intersects the display (110) is referred to as a first point of view (210_1), and
the at least one processor (140) is further configured to identify, based on a change in the gaze direction (201_2) determined by the eye-tracking sensor (120), a second point of view (210_2) where the changed gaze (201_2) of the user intersects the display (110), and change the position on the display (110) at which the tangent screen (111_2) is displayed, such that the center (112_2) of the tangent screen corresponds to the identified second point of view (210_2).

3. The head-mounted display device (100) of claim 2, wherein the at least one processor (140) is further configured to, after displaying the tangent screen (111_1) on the display (110) such that the center (112_1) of the tangent screen corresponds to the first point of view (210_1), determine whether the gaze direction (201_1) of the user (200), which is obtained via the eye-tracking sensor (120) at preset tracking intervals, has changed.

4. The electronic device (100) of any one of claims 1 to 3, wherein the at least one processor (140) is further configured to display at least one point (113_1 or 113_2) on the tangent screen (111_1 or 111_2), receive, from the user, a user input comprising information indicating that the displayed at least one point (113_1 or 113_2) is perceived abnormally, and measure the size and the shape of the scotoma (117) of the user (200), based on the received user input.

5. The electronic device (100) of claim 4, wherein the at least one processor (140) is further configured to change a position of the at least one point (113_1 or 113_2) displayed on the tangent screen (111_1 or 111_2), according to a random pattern (114_1).

6. The electronic device (100) of claim 4, wherein the at least one processor (140) is further configured to change a position of the at least one point (113_1 or 113_2) displayed on the tangent screen (111_1 or 111_2), according to a preset position pattern (114_2).

7. The electronic device (100) of claim 4, wherein the at least one processor (140) is further configured to receive an external input comprising information about a position at which the at least one point (113_1 or 113_2) is to be displayed on the tangent screen (111_1 or 111_2), calculate a position of the at least one point (113_1 or 113_2) to be displayed on the tangent screen (111_1 or 111_2), based on the received external input, and display the at least one point (113_1 or 113_2) at the calculated position.

8. The electronic device (100) of any one of claims 4 to 7, wherein the at least one processor (140) is further configured to display a shape of the at least one point (113_1 or 113_2) such that the shape corresponds to any one of a plurality of preset shapes.

9. The head-mounted display device (100) of any one of claims 1 to 8, wherein the at least one processor (140) is further configured to, after measuring the size and the shape of the scotoma (117) of the user (200) via the tangent screen (111_1 or 111_2), display, on the display (110), visual field training content (111_3) for training a visual field of the user (200), based on the measured size and shape of the scotoma (117) of the user (200).

10. The head-mounted display device (100) of claim 9, wherein the at least one processor (140) is further configured to determine a position on the display (110) at which the visual field training content (111_3) is to be displayed, such that a center (112_3 or 112_4) of the visual field training content corresponds to the identified point of view (210_1 or 210_2), and display the visual field training content (111_3) at the determined position.

11. An operating method of a head-mounted display device (100) comprising a display (110), the operating method comprising:
obtaining information about a gaze direction (201_1 or 201_2) of a user (S100);
identifying, based on the obtained gaze direction (201_1 or 201_2) of the user, a point of view (210_1 or 210_2) where a gaze of the user (200) intersects the display (110) (S200);
determining a position on the display (110) at which a tangent screen (111_1 or 111_2) is to be displayed, such that a center (112_1 or 112_2) of the tangent screen for measuring a size and a shape of a scotoma (117) of the user corresponds to the identified point of view (210_1 or 210_2) (S250); and
displaying the tangent screen (111_1 or 111_2) at the determined position (S300).

12. The operating method of claim 11, wherein the point of view identified in the identifying of the point of view where the gaze of the user (200) intersects the display (110) (S200) is referred to as a first point of view (210_1), and
the operating method further comprises:
after the displaying of the tangent screen (111_1) at the determined position such that the center (112_1) of the tangent screen corresponds to the first point of view (210_1) (S300),
determining whether the gaze direction (201_2) of the user has changed (S310);
identifying, based on the changed gaze direction (201_2) of the user, a second point of view (210_2) where the changed gaze of the user intersects the display (110); and
changing the position on the display (110) at which the tangent screen (111_2) is displayed, such that the center (112_2) of the tangent screen corresponds to the identified second point of view (210_2) (S320).

13. The operating method of claim 12, wherein, in the determining of whether the gaze direction (201_2) of the user has changed (S310), whether the gaze direction (201_1 or 201_2) of the user (200) has changed is determined at preset tracking intervals.

14. The operating method of any one of claims 11 to 13, further comprising:
displaying at least one point (113_1 or 113_2) on the tangent screen (111_1 or 111_2) (S301);
receiving, from the user (200), a user input comprising information indicating that the displayed at least one point (113_1 or 113_2) is perceived abnormally (S400); and
measuring the size and the shape of the scotoma (117) of the user (200), based on the received user input (S500).

15. A computer-readable recording medium having recorded thereon a program for causing a computer to execute the method of any one of claims 11 to 14.
